# EUROPÄISCHE PATENTANMELDUNG

(11) **EP 2 465 941 A1**
(43) Veröffentlichungstag der Anmeldung: **20.06.2012**
(21) Anmeldenummer: 10195869.2
(22) Anmeldetag: 20.12.2010
(51) Int. Cl.: C12Q 1/56

(54) **Verfahren zur simultanen Bestimmung mehrerer Gerinnungsproteasen**

(71) Anmelder: Siemens Healthcare Diagnostics Products GmbH, 35041 Marburg (DE)
(72) Erfinder: Zander,Dr.Norbert, 35039, Marburg (DE)

(57) **Zusammenfassung**

Die vorliegende Erfindung betrifft ein chromogenes Verfahren zur simultanen Bestimmung der Aktivität mehrerer Gerinnungsproteasen beziehungsweise zur simultanen Bestimmung der Inhibition mehrerer Gerinnungsproteasen in einem einzigen Testansatz. Dazu werden zwei chromogene Substrate mit unterschiedlichen Absorptionsmaxima verwendet, deren Farbsignale spektral trennbar sind.

## Beschreibung

Die vorliegende Erfindung liegt auf dem Gebiet der Gerinnungsdiagnostik und betrifft ein Verfahren zur simultanen Bestimmung der Aktivität mehrerer Gerinnungsproteasen beziehungsweise zur simultanen Bestimmung der Inhibition mehrerer Gerinnungsproteasen.

Gängige Antikoagulanzientherapien zielen in erster Linie auf die Hemmung der prokoagulatorischen Gerinnungsfaktoren Thrombin (Faktor IIa) und Faktor Xa. Unterschieden wird zwischen oraler Antikoagulation mit Vitamin K-Antagonisten, wie beispielsweise Coumadin, wodurch eine Hemmung der Gerinnungsfaktorsynthese bewirkt wird, und Antikoagulation durch Hemmung der aktiven Gerinnungsfaktoren im Blutstrom. Bei den Antikoagulanzien, welche aktive Gerinnungsfaktoren im Blutstrom inhibieren bzw. inaktivieren, unterscheidet man Antikoagulanzien mit direkter und indirekter Wirkung. Antikoagulanzien mit direkter Wirkung, wie z.B. Rivaroxaban, Dabigatran oder Melagatran binden an Thrombin oder Faktor Xa und sind damit hochspezifisch. Antikoagulanzien mit indirekter Wirkung, wie z.B. Heparine, binden an endogene Gerinnungsfaktorinhibitoren, wie z.B. Antithrombin, und verstärken deren antikoagulatorische Wirkung um ein Vielfaches.

Alle Antikoagulanzien, welche aktive Gerinnungsfaktoren im Blutstrom inhibieren, zeichnen sich durch ein spezifisches Inaktivierungsmuster aus. Bestimmte Substanzklassen, wie z.B. unfraktionierte, hochmolekulare Heparine, hemmen sowohl Thrombin als auch Faktor Xa. Andere Substanzen wirken hochspezifisch, hemmen also entweder Thrombin (z.B. Hirudin, Dabigatran, Melagatran) oder Faktor Xa (z.B. Pentasaccharide wie Fondaparinux, Rivaroxaban).

Im Laufe der Behandlung einer thromboembolischen Erkrankung wird bisweilen das Antikoagulanz geändert. Klassisch ist die Überleitung von Heparin (Hemmung von Thrombin und Faktor Xa im Blutstrom) auf Coumadin (Hemmung der Gerinnungsfaktorsynthese in der Leber) bei der Behandlung tiefer Beinvenenthrombosen. Bei solchen Änderungen der Therapie kann sich die relative Inaktivierung von Thrombin und Faktor Xa im Blutstrom ändern. Für die Steuerung der Therapie und die Dosierung der Medikamente ist es wichtig, die Aktivität bzw. die Hemmung von Thrombin und von Faktor Xa zu kennen. Es ist daher notwendig, die Aktivität bzw. die Inhibition der aktiven Gerinnungsfaktoren Thrombin und Faktor Xa im Blut eines Patienten zuverlässig bestimmen zu können.

In der Gerinnungsdiagnostik werden sogenannte Globalteste zur Untersuchung der Funktionalität der Blutgerinnungskaskade und sogenannte Einzelteste zur Bestimmung der Aktivität einzelner Blutgerinnungsfaktoren unterschieden. Sowohl für die Globalteste als auch für die Einzelteste sind unterschiedliche Testformate bekannt. In Bezug auf das Testformat werden im wesentlichen Gerinnungsteste und chromogene Teste unterschieden.

Bei einem chromogenen Test wird die zu untersuchende Patientenprobe, die üblicherweise aus Plasma besteht, mit einem Gerinnungsaktivator und mit einem Substrat für einen Gerinnungsfaktor vermischt. Da es sich bei den meisten Blutgerinnungsfaktoren um Serin-Endopeptidasen handelt, also um Hydrolasen, die Peptidbindungen spalten können, werden vorwiegend Peptidsubstrate verwendet, die möglichst spezifisch von dem zu bestimmenden Blutgerinnungsfaktor gespalten werden und die eine nachweisbare Signalgruppe aufweisen. Bevorzugterweise werden abspaltbare chromogene oder fluorogene Signalgruppen verwendet, die photometrisch bestimmt werden. In den Patentdokumenten EP 0034122 A1 und US 4,508,644 sind eine Vielzahl von chromogenen Peptidsubstraten und deren Verwendung in gerinnungsdiagnostischen Tests beschrieben, z.B. zur Bestimmung der proteolytischen Gerinnungsfaktoren Faktor IIa (Thrombin) und Xa. In dem Dokument EP 0078764 A1 ist ein chromogenes Verfahren zur Bestimmung des proteolytischen Gerinnungsfaktors XIIa beschrieben.

Insbesondere mit Hilfe der chromogenen Teste können auch Antikoagulanzien, die die Aktivität von Blutgerinnungsfaktoren inhibieren, in Patientenproben bestimmt werden. Dazu wird die zu untersuchende Patientenprobe üblicherweise mit einem aktivierten Gerinnungsfaktor und mit einem Substrat für diesen Gerinnungsfaktor vermischt. Je mehr Antikoagulanz in der Probe enthalten ist, desto stärker wird der aktivierte Gerinnungsfaktor inhibiert und desto weniger Substrat wird gespalten.

Bei den etablierten, auch kommerziell erhältlichen chromogenen Testen werden insbesondere die Chromophore para-Nitroanilin (pNA) und 5-Amino-2-Nitro-Benzoesäure (ANBA), welche ein Absorptionsmaximum bei 405 nm aufweisen, verwendet. Die entstehende gelbe Farbe wird in der Regel photometrisch bestimmt. Bei der Bestimmung von Antikoagulanzien verhält sich die Farbkonzentration im Testansatz umgekehrt proportional zur Antikoagulanzkonzentration in der Probe.

Um die Hemmung von Thrombin und Faktor Xa bestimmen zu können, ist es notwendig, zwei getrennte Teste durchzuführen, in denen jeweils die Inhibition eines der beiden Gerinnungsfaktoren bestimmt wird. Wünschenswert wäre die simultane Bestimmung der Aktivität bzw. Hemmung beider Gerinnungsfaktoren in einem einzigen Testansatz. Dies hätte die Vorteile, dass der Material- und Zeitaufwand verringert werden würden und dass beide Bestimmungen unter denselben Bedingungen durchgeführt werden könnten, wodurch Abweichungen bei der Testdurchführung, wie z.B. Pipettierfehler, die dazu führen könnten, dass eine Fehlbestimmung der Relation der beiden Ergebnisse entstehen könnte, vermieden werden.

Der vorliegenden Erfindung lag daher die Aufgabe zugrunde ein Verfahren bereit zu stellen, das die simultane Bestimmung von Thrombin und Faktor Xa in einem einzigen Testansatz ermöglicht. In der WO 2006/072602 A1 ist ein Verfahren zur simultanen Bestimmung von Thrombin und Plasmin, einem Enzym des fibrinolytischen Systems, beschrieben, wobei fluoreszente Substrate verwendet werden.

Die der Erfindung zugrundeliegende Aufgabe wird dadurch gelöst, dass eine Probe mit einem ersten chromogenen Substrat mit Spezifität für den ersten proteolytischen Gerinnungsfaktor und mit einem zweiten chromogenen Substrat mit Spezifiät für den zweiten proteolytischen Gerinnungsfaktor vermischt wird, wobei das erste chromogene Substrat ein Chromophor aufweist, dessen Absorptionsmaximum mindestens 100 nm vom Absorptionsmaximum des Chromophors des zweiten chromogenen Substrats abweicht. Die entstehenden chromogenen Signale sind spektral trennbar und können bei unterschiedlichen Wellenlängen photometrisch unabhängig voneinander bestimmt werden.

Ein Gegenstand der vorliegenden Erfindung ist damit ein Verfahren zur simultanen Bestimmung der Aktivität eines ersten und eines zweiten proteolytischen Gerinnungsfaktors in einem einzigen Testansatz, wobei eine Probe mit einem ersten chromogenen Substrat mit Spezifität für den ersten proteolytischen Gerinnungsfaktor und mit einem zweiten chromogenen Substrat mit Spezifität für den zweiten proteolytischen Gerinnungsfaktor vermischt wird und wobei die Farbsignalentstehung photometrisch bestimmt wird und wobei das erste chromogene Substrat ein Chromophor aufweist, dessen Absorptionsmaximum mindestens 100 nm vom Absorptionsmaximum des Chromophors des zweiten chromogenen Substrats abweicht. Unter dem Begriff "proteolytischer Gerinnungsfaktor" ist jede plasmatische Serinprotease zu verstehen, die eine prokoagulatorische (gerinnungsfördernde), antikoagulatorische (gerinnungshemmende) oder fibrinolytische (gerinnselauflösende) Funktion im Blutgerinnungssystem eines Säugetiers, bevorzugt des Menschen, hat. Prokoagulatorische proteolytische Gerinnungsfaktoren sind beispielsweise Faktor IIa (Thrombin), Faktor VIIa, Faktor IXa, Faktor Xa, Faktor XIa und Faktor XIIa. Ein antikoagulatorischer proteolytischer Gerinnungsfaktor ist beispielsweise Protein Ca (aktiviertes Protein C). Ein fibrinolytischer proteolytischer Gerinnungsfaktor ist beispielsweise Plasmin.

Unter dem Begriff "simultane Bestimmung" ist die Bestimmung der beiden proteolytischen Gerinnungsfaktoren in einem einzigen Testansatz zu verstehen.

Die Messung der Absorption des Testansatzes über die Zeit kann durch in kurzen Zeitabständen abwechselnden Pulsen von Licht der Wellenlängen, die den Absorptionsmaxima der beiden verwendeten Chromophore entsprechen, erfolgen. Alternativ kann der Testansatz mit weißem Licht bestrahlt werden, das nach Durchgang durch den Testansatz spektral zerlegt wird.

Unter einer "Probe" ist im Sinne der Erfindung das Material zu verstehen, das die nachzuweisenden proteolytischen Gerinnungsfaktoren bzw. das oder die zu bestimmenden Antikoagulanzien vermutlich enthält. Der Begriff Probe umfasst insbesondere menschliche oder tierische Körperflüssigkeiten, vornehmlich Blut und Plasma.

Unter einem "chromogenen Substrat mit Spezifität für einen proteolytischen Gerinnungsfaktor" ist ein Substrat zu verstehen, das hinreichend spezifisch von einem proteolytischen Gerinnungsfaktor umgesetzt wird, wobei bei der spezifischen Substratumsetzung ein Chromophor freigesetzt wird. Insbesondere spaltbare Substrate, welche mindestens eine Spaltstelle für einen aktivierten Gerinnungsfaktor aufweisen, sind dem Fachmann hinlänglich bekannt. Ein spaltbares Substrat kann ein synthetisch, rekombinant oder biotechnologisch hergestelltes Molekül oder ein natürliches Molekül sein, das durch Einwirken des aktivierten proteolytischen Gerinnungsfaktors in zwei Spaltprodukte zerlegt wird. Ein spaltbares Substrat kann ganz oder teilweise aus einem Peptid bestehen. Bevorzugterweise umfasst es zumindest im Bereich der Spaltstelle einen Peptidanteil. Bevorzugterweise besteht der Peptidanteil eines spaltbaren Substrats aus 3 bis etwa 150 Aminosäureresten. In den Patentdokumenten EP 0034122 A1 und US 4,508,644 sind eine Vielzahl von chromogenen Peptidsubstraten, deren Herstellung und deren Verwendung in gerinnungsdiagnostischen Tests beschrieben, z.B. zur Bestimmung der Gerinnungsfaktoren Faktor IIa (Thrombin) und Xa. In dem Dokument EP 78764 A1 ist ein chromogenes Verfahren zur Bestimmung des Gerinnungsfaktors XIIa beschrieben.

Unter einem "Chromophor" ist eine Signalgruppe ("Label") zu verstehen, das von dem Substrat durch Einwirkung eines spezifischen proteolytischen Gerinnungsfaktors abspaltbar ist und das nach Abspaltung vom Substrat andere optische Eigenschaften aufweist als im ungespaltenen Zustand. Erfindungsgemäß weist das erste chromogene Substrat ein Chromophor auf, dessen Absorptionsmaximum mindestens 100 nm vom Absorptionsmaximum des Chromophors des zweiten chromogenen Substrats abweicht. Tabelle 1 zeigt eine Auswahl bekannter Chromophore, deren Absorptionsmaxima nach Abspaltung sowie bevorzugte Kombinationen von Chromophoren, deren Absorptionsmaxima mindestens 100 nm voneinander abweichen.

**Tabelle 1**

| **Chromophor** | **Absorptions-maximum** | **Kombinierbar mit** |
|---|---|---|
| para-Nitroanilin (pNA) | 405 nm | Colour Index Basic Blue 49 oder 124 |
| 5-Amino-2-Nitro-Benzoesäure (ANBA) | 405 nm | Colour Index Basic Blue 49 oder 124 |
| Colour Index Basic Blue 49 | 625 nm | pNA, ANBA |
| Colour Index Basic Blue 124 | 625 nm | pNA, ANBA |

Besonders bevorzugte Chromophore sind Phenoxazimderivate, wie z.B. Colour Index Basic Blue 49 (C.I. Basic Blue 49, CAS Registrierungsnummer 11075-19-7) oder Colour Index Basic Blue 124 (C.I. Basic Blue 124, CAS Registrierungsnummer 89106-91-2), die ein Absorptionsmaximum von etwa 600 nm aufweisen und eine Blaufärbung bewirken. In dem Patentdokument EP 0258784 A2 sind besonders bevorzugte Phenoxazimderivate bzw. entsprechend gelabelte Peptidsubstrate offenbart.

Das erfindungsgemäße Verfahren kann zur simultanen Bestimmung der Aktivität von zwei proteolytischen Gerinnungsfaktoren, die in der Patientenprobe enthalten sind, verwendet werden, um den Gerinnungsstatus eines Patienten zu untersuchen. Dazu wird die Probe üblicherweise vor der Zugabe der beiden chromogenen Substrate mit einem oder mehreren Agenzien vermischt, die eine direkte oder indirekte Aktivierung der zu bestimmenden proteolytischen Gerinnungsfaktoren bewirken. Unter direkter Aktivierung ist zu verstehen, dass ein Agens verwendet wird, das den zu bestimmenden proteolytischen Gerinnungsfaktor direkt aktiviert, unabhängig von der Anwesenheit anderer Gerinnungsfaktoren. Unter indirekter Aktivierung ist zu verstehen, dass ein Agens verwendet wird, das einen oder mehrere Blutgerinnungsfaktoren der Blutgerinnungskaskade aktiviert, welche wiederum den zu untersuchenden proteolytischen Gerinnungsfaktor aktivieren.

Die Art des Agens hängt davon ab, welcher Gerinnungsfaktor bestimmt werden soll, ob die Aktivität des Gerinnungsfaktors allein bestimmt werden soll oder ob die Funktionalität der Blutgerinnungskaskade oder eines Teilbereichs der Blutgerinnungskaskade (extrinsischer oder intrinsischer Weg) anhand eines Gerinnungsfaktors bestimmt werden soll. Substanzen und spezifische Mischungen verschiedener Substanzen, die eine direkte oder indirekte Aktivierung von proteolytischen Gerinnungsfaktoren ermöglichen, sind dem Fachmann hinlänglich bekannt und umfassen beispielsweise Phospholipide, wie z.B. negativ geladene Phospholipide; Lipoproteine, wie z.B. Thromboplastin; Proteine, wie z.B. Gewebefaktor, aktivierte Serinproteasen, wie z.B. Faktor IIa (Thrombin), Faktor VIIa, Faktor IXa, Faktor Xa, Faktor XIa, Faktor XIIa oder aktiviertes Protein C; Schlangengifte, wie z.B. PROTAC® Enzym, Ecarin, Textarin, Noscarin, Batroxobin, Thrombocytin oder Russell's Viper Venom (RVV); Kontaktaktivatoren, wie z.B. Silica, Kaolin, Ellagsäure oder Celite. Weitere Substanzen, die ein Aktivierungsagens enthalten kann, sind z.B. Puffersubstanzen, Salze, Detergenzien, Ionen, insbesondere Calciumionen und Chelatbildner.

In einer Ausführungsform, kann dem Testansatz ferner ein Inhibitor der Fibrinaggregation zugegeben werden. Unter einem Fibrinaggregationsinhibitor ist eine Substanz, insbesondere ein synthetisches Oligopeptid, zu verstehen, das die Aneinanderlagerung (Polymerisation) der Fibrinmonomere, die unter der Einwirkung von Thrombin entstehen, inhibiert und damit eine Gerinnselbildung, die die photometrische Bestimmung der Farbsignale beeinträchtigen könnte, im Reaktionsgemisch verhindert (siehe z.B. EP 0456152 B1).

Weiterhin kann das erfindungsgemäße Verfahren zur simultanen Bestimmung der Aktivität von zwei proteolytischen Gerinnungsfaktoren, die der Patientenprobe zugegeben wurden, verwendet werden, um das antikoagulatorische Potenzial eines Patienten zu untersuchen. Dazu wird die Patientenprobe mit definierten Mengen von mindestens zwei prokoagulatorischen proteolytischen Gerinnungsfaktoren und mit den beiden chromogenen Substraten mit Spezifität für die zugesetzten Gerinnungsfaktoren vermischt, und die Hemmung der proteolytischen Aktivität der Gerinnungsfaktoren wird bestimmt. Je größer das antikoagulatorische Potenzial des Patienten ist, d.h. je mehr Antikoagulanz in der Probe enthalten ist, desto stärker wird/werden der/die aktivierte(n) prokoagulatorischen Gerinnungsfaktor(en) inhibiert und desto weniger Substrat wird gespalten. Die Hemmung der proteolytischen Aktivität der Gerinnungsfaktoren kann durch den Vergleich mit einem Kontrolltestansatz quantifiziert werden, bei dem eine Normalprobe, die kein Antikoagulanz enthält, z.B. humanes Normalplasma, als Probe verwendet wird.

Welche aktivierten Gerinnungsfaktoren zugegeben werden, hängt davon ab, welche Antikoagulanzien bestimmt werden sollen.

Für die Bestimmung eines Heparins, d.h. eines hochmolekularen, unfraktionierten Heparins (HMW Heparin) oder eines niedermolekularen Heparins (LMW Heparin) oder eines Heparinoids, eignet sich insbesondere die Zugabe von Faktor IIa (Thrombin) oder von Faktor Xa. Für die Bestimmung eines direkten Thrombininhibitors, z.B. von Argatroban, Melagatran, Ximelagatran, Bivalirudin, Dabigatran oder Hirudin, eignet sich insbesondere die Zugabe von Faktor IIa (Thrombin). Für die Bestimmung eines direkten Faktor Xa-Inhibitors, z.B. von Rivaroxaban, eignet sich insbesondere die Zugabe von Faktor Xa.

Die vorliegende Erfindung betrifft weiterhin ein Testkit zur Bestimmung des antikoagulatorischen Potenzials einer Patientenprobe. Ein erfindungsgemäßes Testkit umfasst ein erstes Reagenz mit einer definierten Konzentration eines ersten proteolytischen Gerinnungsfaktors, und ein zweites Reagenz mit einer definierten Konzentration eines zweiten proteolytischen Gerinnungsfaktors, und
a) ein drittes Reagenz enthaltend ein erstes chromogenes Substrat mit Spezifität für den ersten proteolytischen Gerinnungsfaktor und ein zweites chromogenes Substrat mit Spezifität für den zweiten proteolytischen Gerinnungsfaktor; oder
b) ein drittes Reagenz enthaltend ein erstes chromogenes Substrat mit Spezifität für den ersten proteolytischen Gerinnungsfaktor und ein viertes Reagenz enthaltend ein zweites chromogenes Substrat mit Spezifität für den zweiten proteolytischen Gerinnungsfaktor,
wobei das erste chromogene Substrat ein Chromophor aufweist, dessen Absorptionsmaximum mindestens 100 nm vom Absorptionsmaximum des Chromophors des zweiten chromogenen Substrats abweicht.

In einem bevorzugten Testkit weist das erste chromogene Substrat mit Spezifität für den ersten proteolytischen Gerinnungsfaktor ein Chromophor aus der Gruppe para-Nitroanilin und 5-Amino-2-Nitro-Benzoesäure auf, und das zweite chromogene Substrat mit Spezifität für den zweiten proteolytischen Gerinnungsfaktor weist ein Chromophor aus der Gruppe der Phenoxazimderivate auf, oder umgekehrt.

Ein besonders bevorzugtes Testkit umfasst ein erstes Reagenz mit einer definierten Thrombinkonzentration und ein zweites Reagenz mit einer definierten Faktor Xa-Konzentration und mindestens ein weiteres Reagenz enthaltend ein Thrombinspezifisches und/oder ein Faktor Xa-spezifisches chromogenes Substrat, wobei das Thrombin-spezifische chromogene Substrat ein Chromophor aufweist, dessen Absorptionsmaximum mindestens 100 nm vom Absorptionsmaximum des Chromophors des Faktor Xa-spezifischen chromogenen Substrats abweicht. Die beiden Substrate können in einem einzigen Reagenz enthalten sein oder in getrennten Reagenzien vorliegen. Bevorzugterweise weist das Thrombin-spezifische chromogene Substrat ein Chromophor aus der Gruppe para-Nitroanilin und 5-Amino-2-Nitro-Benzoesäure auf, und das Faktor Xa-spezifische chromogene Substrat weist ein Chromophor aus der Gruppe der Phenoxazimderivate auf, oder umgekehrt.

Die Reagenzien des erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

### FIGURENBESCHREIBUNG

**Figur 1**
   Balkendiagramm der Inhibition der Faktor Xa-Aktivität [%] in Normalplasmaproben angereichert mit Rivaroxaban und/oder Argatroban in verschiedenen Konzentrationen (siehe Beispiel 2). Gemessen wurde die Spaltung des Faktor Xa-spezifischen Peptidsubstrats mit ANBA-Chromophor bei einer Wellenlänge von 405 nm. Unabhängig von der Argatroban-Konzentration (Thrombininhibitor) wird in Proben mit erhöhter Rivaroxaban-Konzentration (Faktor Xa-Inhibitor) eine gesteigerte Faktor Xa-Inhibition gemessen.
**Figur 2**
   Balkendiagramm der Inhibition der Thrombinaktivität [%] in Normalplasmaproben angereichert mit Rivaroxaban und/oder Argatroban in verschiedenen Konzentrationen (siehe Beispiel 2). Gemessen wurde die Spaltung des Thrombin-spezifischen Peptidsubstrats mit Basic Blue 49-Chromophor bei einer Wellenlänge von 575 nm. Unabhängig von der Rivaroxaban-Konzentration (Faktor Xa-Inhibitor) wird in Proben mit erhöhter Argatroban-Konzentration (Thrombininhibitor) eine gesteigerte Thrombin-Inhibition gemessen.

Die folgenden Ausführungsbeispiele dienen der Veranschaulichung des erfindungsgemäßen Verfahrens und sind nicht als Einschränkung zu verstehen.

### BEISPIELE

### Beispiel 1: Simultane Bestimmung der Thrombin- und der Faktor Xa-Aktivität in Blutplasma in einem einzigen Reaktionsansatz

Folgende Komponenten wurden zu einem Reaktionsansatz vermischt:

| | | |
|---|---|---|
| 10 | µL | Probe |
| 10 | µL | Humaner Normalplasmapool |
| 40 | µL | Substratreagenz (4 mM Z-D-Leu-Gly-Arg-ANBA-Methylamid, 0,8 mM Tos-Gly-Pro-Arg-Basic Blue 49 in Mannitolpuffer, pH 4.0) |
| 100 | µL | Faktor Xa-Reagenz (0,75 U/mL humaner Faktor Xa in 4,5 g/L TRIS, 9 g/L NaCl, 0,56 g/L EDTA, pH 8.0) |
| 100 | µL | Thrombin-Reagenz (5 U/mL bovines Thrombin in 1,2 g/L TRIS, pH 8.2) |

Als Probe wurde humanes Normalplasma verwendet. Faktor Xa spaltet ANBA von dem Faktor Xa-spezifischen Peptidsubstrat Z-D-Leu-Gly-Arg-ANBA-Methylamid ab, was eine Zunahme der optischen Dichte über die Zeit im Reaktionsansatz bei einer Wellenlänge von 405 nm bewirkt. Gleichzeitig spaltet Thrombin Basic Blue 49 von dem Thrombin-spezifischen Peptidsubstrat Tos-Gly-Pro-Arg-Basic Blue 49 ab, was eine Zunahme der optischen Dichte über die Zeit im Reaktionsansatz bei einer Wellenlänge von 575 nm bewirkt.

Die Messungen der optischen Dichten des Reaktionsansatzes bei 405 nm und 575 nm sowie die Auswertung der Reaktionskinetiken wurden in einem vollautomatischen Sysmex® CS-2000i Gerinnungsanalyzer simultan durchgeführt. Zur Messung der optischen Dichten wurde der Reaktionsansatz abwechselnd mit Licht der vorgenannten Wellenlängen bestrahlt, und die Extinktion des Lichts, verursacht durch die Färbung des Reaktionsansatzes, wurde wellenlängen- und zeitabhängig bestimmt.

Die Steigungen der Reaktionskinetiken korrelieren mit der jeweiligen Enzymaktivität. Für eine Normalplasmaprobe betrug die Faktor Xa-spezifische Extinktionsänderung bei 405 nm 0,236 pro Minute, während die thrombinspezifische Extinktionsänderung bei 575 nm 0,111 pro Minute betrug.

### Beispiel 2: Simultane Bestimmung von Thrombin- und Faktor Xa-Inhibitoren in einem einzigen Reaktionsansatz

Normalplasma wurde mit verschiedenen Konzentrationen von Rivaroxaban, einem spezifischen Faktor Xa-Inhibitor, und/ oder von Argatroban, einem spezifischen Thrombininhibitor, angereichert und in einem Verfahren gemäß Beispiel 1 als Probe eingesetzt.

Die Steigungen der Reaktionskinetiken für Normalplasma ohne Inhibitoren wurden als 100 % der jeweiligen Enzymaktivität definiert. Die Ergebnisse der Plasmaproben mit verschiedenen Inhibitorkonzentrationen wurden anhand dieser Referenz ausgewertet. Die Ergebnisse (Inhibition in %) sind in Tabelle 2 zusammengefasst.

**Tabelle 2**

| **Rivaroxaban (ng/mL)** | **Argatroban (ng/mL)** | **F Xa-Inhibition [%]** | **Thrombin-Inhibition [%]** |
|---|---|---|---|
| 0 | 0 | 2 | 0 |
| 0 | 375 | 0 | 18 |
| 0 | 750 | 0 | 31 |
| 125 | 0 | 42 | 0 |
| 125 | 375 | 40 | 23 |
| 125 | 750 | 39 | 33 |
| 250 | 0 | 65 | 5 |
| 250 | 375 | 62 | 22 |
| 250 | 750 | 63 | 35 |

Die Ergebnisse zeigen, dass die Verwendung von zwei chromogenen Substraten mit unterschiedlicher Enzymspezifität und mit Chromophoren, die unterschiedliche Absorptionsmaxima aufweisen, die simultane, unabhängige Bestimmung von Inhibitoren mit Spezifität für unterschiedliche Gerinnungsfaktoren in einem einzigen Reaktionsansatz ermöglicht.

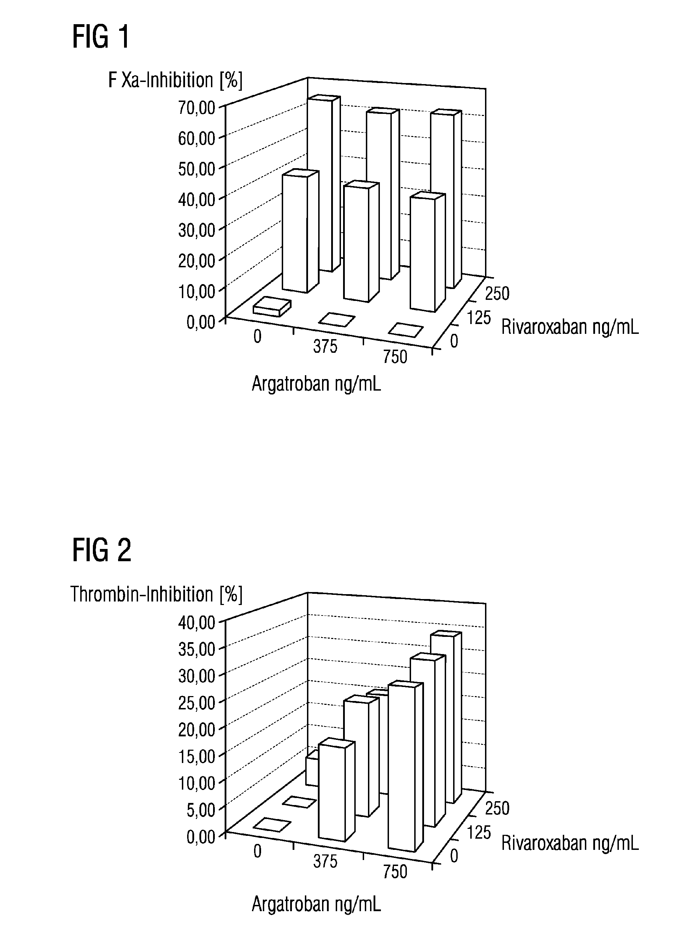

## Patentansprüche

1. Verfahren zur simultanen Bestimmung der Aktivität eines ersten und eines zweiten proteolytischen Gerinnungsfaktors in einem einzigen Testansatz, wobei eine Probe mit einem ersten chromogenen Substrat mit Spezifität für den ersten proteolytischen Gerinnungsfaktor und mit einem zweiten chromogenen Substrat mit Spezifität für den zweiten proteolytischen Gerinnungsfaktor vermischt wird und wobei die Farbsignalentstehung photometrisch bestimmt wird, **dadurch gekennzeichnet, dass** das erste chromogene Substrat ein Chromophor aufweist, dessen Absorptionsmaximum mindestens 100 nm vom Absorptionsmaximum des Chromophors des zweiten chromogenen Substrats abweicht.

2. Verfahren nach Anspruch 1, wobei der erste und der zweite proteolytische Gerinnungsfaktor ausgewählt sind aus der Gruppe Thrombin, Faktor VIIa, Faktor IXa, Faktor Xa, Faktor XIa, Faktor XIIa, Protein Ca und Plasmin.

3. Verfahren nach einem der vorhergehenden Ansprüche, wobei der erste oder zweite proteolytische Gerinnungsfaktor Thrombin ist.

4. Verfahren nach einem der vorhergehenden Ansprüche, wobei der erste oder zweite proteolytische Gerinnungsfaktor Faktor Xa ist.

5. Verfahren nach einem der vorhergehenden Ansprüche, wobei das erste oder das zweite chromogene Substrat para-Nitroanilin oder 5-Amino-2-Nitro-Benzoesäure als Chromophor aufweist.

6. Verfahren nach einem der vorhergehenden Ansprüche, wobei das erste oder das zweite chromogene Substrat ein Phenoxazimderivat als Chromophor aufweist.

7. Verfahren nach einem der vorhergehenden Ansprüche, wobei der erste und der zweite proteolytische Gerinnungsfaktor in der Probe enthalten sind und durch Zugabe eines oder mehrerer Agenzien zur Aktivierung der Gerinnungsfaktoren aktiviert werden.

8. Verfahren nach einem der Ansprüche 1 bis 6, wobei definierte Mengen des ersten und des zweiten proteolytischen Gerinnungsfaktors mit der Probe vermischt werden und wobei anhand der Hemmung der Farbsignalentstehung die Antikoagulanzkonzentration in der Probe bestimmt wird.

9. Testkit umfassend ein erstes Reagenz mit einer definierten Konzentration eines ersten proteolytischen Gerinnungsfaktors, und ein zweites Reagenz mit einer definierten Konzentration eines zweiten proteolytischen Gerinnungsfaktors, und
a. ein drittes Reagenz enthaltend ein erstes chromogenes Substrat mit Spezifität für den ersten proteolytischen Gerinnungsfaktor und ein zweites chromogenes Substrat mit Spezifität für den zweiten proteolytischen Gerinnungsfaktor; oder
b. ein drittes Reagenz enthaltend ein erstes chromogenes Substrat mit Spezifität für den ersten proteolytischen Gerinnungsfaktor und ein viertes Reagenz enthaltend ein zweites chromogenes Substrat mit Spezifität für den zweiten proteolytischen Gerinnungsfaktor,
wobei das erste chromogene Substrat ein Chromophor aufweist, dessen Absorptionsmaximum mindestens 100 nm vom Absorptionsmaximum des Chromophors des zweiten chromogenen Substrats abweicht.

10. Testkit nach Anspruch 9, wobei der erste proteolytische Gerinnungsfaktor Thrombin ist.

11. Testkit nach einem der Ansprüche 9 oder 10, wobei der zweite proteolytische Gerinnungsfaktor Faktor Xa ist.

12. Testkit nach einem der Ansprüche 9 bis 11, wobei das erste chromogene Substrat mit Spezifität für den ersten proteolytischen Gerinnungsfaktor ein Chromophor aus der Gruppe para-Nitroanilin und 5-Amino-2-Nitro-Benzoesäure aufweist und das zweite chromogene Substrat mit Spezifität für den zweiten proteolytischen Gerinnungsfaktor ein Chromophor aus der Gruppe der Phenoxazimderivate aufweist, oder umgekehrt.
